# EUROPÄISCHE PATENTSCHRIFT

(11) **EP 4 122 398 B1**
(45) Veröffentlichungstag und Bekanntmachung des Hinweises auf die Patenterteilung: **02.07.2025**
(21) Anmeldenummer: 21187402.9
(22) Anmeldetag: 23.07.2021
(51) Int. Cl.: A61B 6/04

(54) **VERFAHREN FÜR EIN BEWEGEN EINER PATIENTENLIEGE**
METHOD FOR MOVING A PATIENT BED
PROCÉDÉ DE DÉPLACEMENT D'UN LIT DE PATIENT

(43) Veröffentlichungstag der Anmeldung: 25.01.2023
(73) Patentinhaber: Siemens Healthineers AG, 91301 Forchheim (DE)
(72) Erfinder: Baumann, Berthold, 95506 Kastl (DE); Krämer, Alexander, 92699 Irchenrieth (DE); Wurzer, Gerhard, 92708 Mantel (DE)
(74) Vertreter: Siemens Healthineers Patent Attorneys

(56) Entgegenhaltungen:
- EP-A1- 2 675 359
- WO-A1-2021/094391
- JP-A- 2002 058 667
- JP-A- 2009 000 209
- US-A1- 2017 325 759

## Beschreibung

Die Erfindung betrifft ein Verfahren für ein Bewegen einer Patientenliege in Abhängigkeit eines Körperwerts eines auf der Patientenliege für eine medizinische Bildgebung gelagerten Patienten, die zugehörige Patientenliege und das zugehörige Computerprogrammprodukt.

Ein Bewegen einer herkömmlichen Patientenliege innerhalb eines Aufnahmebereichs einer Bildgebungsmodalität erfolgt typischerweise unabhängig von dem auf der Patientenliege gelagerten Patienten. Regelmäßig erfolgt das Bewegen der herkömmlichen Patientenliege, ohne dass der Patient vorher digital erfasst und die Bewegung an den Patienten angepasst wird. Die herkömmliche Patientenliege und deren herkömmliche Antriebskette werden insbesondere auf ein Maximalbeispiel eines beispielhaften Patienten ausgelegt. Das Maximalbeispiel kann insbesondere einen Massewert von 300 oder 400 kg umfassen. Die Bewegung der herkömmlichen Patientenliege erfolgt typischerweise bei einer gemäß diesem Massewert festgelegten unveränderlichen Maximalgeschwindigkeit.

Aus der DE 103 33 295 A1 ist eine Röntgendiagnostikeinrichtung mit einem Röntgenstrahler, einer mit diesem verbundenen Steuervorrichtung, einem Detektor und einem Patientenlagerungstisch bekannt, wobei die Röntgendiagnostikeinrichtung eine Vorrichtung aufweist, die aus der Körperlänge und dem Gewicht des zu untersuchenden Patienten den Body Maß Index errechnet, und wobei die Vorrichtung mit der Steuervorrichtung zur Beeinflussung von die Röntgenstrahlung bestimmenden Parametern verbunden ist.

DE 198 09 738 A1 offenbart ein Röntgendiagnostikgerät, insbesondere für einen digitalen Lungenarbeitsplatz, mit einer Steuereinheit zum Voreinstellen der für eine Aufnahme erforderlichen Blendeneinstellung, die mit einer Recheneinheit versehen ist, die aufgrund von patientenbezogenen Daten eine Berechnung in Hinsicht auf eine dem Patienten angepasste Voreinstellung vornimmt, wobei die Steuereinheit mit einem direkten Zugriff zu den Patientendaten versehen ist und ein Organprogrammspeicher mit Standardwerten der Blendeneinstellung enthält, um aus den patientenbezogenen Daten die Größe des Organs, bzw. einer zu untersuchenden Körperregion, zu ermitteln und motorisch den optimalen Blendenwert einzustellen.

In der WO 2015/081 295 A1 wird ein System und ein Verfahren zur Verbesserung von Projektions-basierten und tomographischen Röntgen beschrieben, welche ein Tiefenerfassungsgerät zur Messung einer Tiefe zumindest eins Körperteils des Patienten mittels des Tiefenerfassungsgeräts und eine Steuereinheit zur Berechnung einer Dicke und/oder eines Umfang des Körperteils unter Verwendung der Tiefeninformation umfassen. Die berechnete Dicken- und/oder Umfanginformation wird dazu verwendet, ein optimales Level an Röntgenstrahlendosis für das Körperteil zu ermitteln. Das System und das Verfahren umfasst auch eine Kamera zur Identifizierung des Körperteils, welches untersucht werden soll, und zur Detektion jeglicher Bewegung des identifizierten Körperteils.

WO 2021/094 391 A1 offenbart ein Steuersystem zum Steuern eines Bildgebungsgeräts, welches eine elektronische Steuereinheit mit einer Steuerspeicher und einer Steueranzeige umfasst. Die elektronische Steuereinheit ist im Betrieb zu einem Steuern des Bildgebungsgeräts verbunden und dazu programmiert, einen Steuerzustand in dem Steuerspeicher zu speichern; eine Bildgebungsgerätsetupsoftware auszuführen, so dass die elektronische Steuereinheit eine Setup-Benutzeroberfläche auf der elektronischen Steuereinheit bereitstellt, mittels welcher ein Nutzer den Steuerzustand in dem Steuerspeicher konfiguriert; und eine Steuersoftware auszuführen, so dass die elektronische Steuereinheit das Bildgebungsgerät für eine Akquisition von Bildern gemäß dem in dem Steuerspeicher gespeicherten Steuerzustand steuert.

Die noch nicht offengelegte DE 10 2020 214 654.3 beschreibt eine Fern-Kommunikation mit einer medizintechnischen Einrichtung mit Hilfe eines digitalen Zwillings, wobei die Fernbedieneinrichtung eine Datenerfassungseinheit zum Erfassen von Zustandsdaten und Maschinendaten von einer medizintechnischen Einrichtung, eine Auswertungseinheit zum Erzeugen eines digitalen Zwillings auf Basis der Zustandsdaten und Maschinendaten, eine Anzeigeeinheit zum Anzeigen des digitalen Zwillings und eine Steuerungseinheit zum Ansteuern der medizintechnischen Einrichtung auf Basis der angezeigten Daten des digitalen Zwillings aufweist.

US 2017/0 325 759 A1 offenbart ein Röntgen-CT Gerät mit einer Patientenliege, auf welcher ein Patient gelagert ist. Eine Recheneinheit steuert eine Antriebskette, um die Patientenliege in einer longitudinalen Richtung bewegen zu können. Die Recheneinheit zeigt ferner eine Information über die dabei auftretenden Vibrationen an.

JP 2009 000 209 A betrifft ein Steuerverfahren eines medizinischen diagnostischen bildgeberischen Geräts und eines Antriebsmittels, welches die benötigte Beschleunigungszeit verkürzt und gleichzeitig Vibrationen unterdrückt.

In JP 2002 058 667 A ist eine medizinische diagnostische bildbasierte Methode mittels eines Röntgen-CT Geräts beschrieben.

EP 2 675 359 A1 bzw. WO 2012/110 982 A1 zeigt einen Patiententräger und eine Patiententrägersteuereinheit zur Steuerung des Patiententrägers auf Basis eines Bewegungsalgorithmus innerhalb eines Bildgebungssystems, wobei der Bewegungsalgorithmus die Vibration des Patiententrägers während der Bildgebung mittels des Bildgebungssystems reduziert.

Der Erfindung liegt die Aufgabe zu Grunde, ein Verfahren für ein Bewegen einer Patientenliege in Abhängigkeit eines Körperwerts eines auf der Patientenliege für eine medizinische Bildgebung gelagerten Patienten, die zugehörige Patientenliege und das zugehörige Computerprogrammprodukt anzugeben, so dass die Antriebskette der Patientenliege patientenabhängig und zustandsabhängig gesteuert wird.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Bewegen einer Patientenliege in Abhängigkeit eines Körperwerts eines auf der Patientenliege für eine medizinische Bildgebung gelagerten Patienten, wobei die medizinische Bildgebung in einer Bildgebungsmodalität mit einem Aufnahmebereich erfolgt, wobei die Patientenliege innerhalb des Aufnahmebereichs mittels einer Antriebskette bewegbar ist, umfasst folgende Schritte:
- Bereitstellen eines Körperwerts, insbesondere einer Masse und/oder einer Statur, des Patienten,
- Einstellen eines Betriebsparameters der Antriebskette mittels einer Steuereinheit für die Steuerung der Antriebskette in Abhängigkeit des Körperwerts derart, dass ein Geschwindigkeitswert der Patientenliege bis zu einem vorgegebenen oder Körperwert-abhängigen Maximalwert erhöht wird, und gegebenenfalls erneutes Einstellen des Betriebsparameters, damit ein Aufschaukeln der Patientenliege bei der Bewegung der Patientenliege zumindest teilweise unterdrückt wird, und
- Bewegen der Patientenliege mittels der Antriebskette gemäß dem erhöhten Geschwindigkeitswert zur Durchführung der medizinischen Bildgebung,
dadurch gekennzeichnet, dass der Körperwert in einen digitalen Zwilling der Antriebskette der Patientenliege eingegeben wird, wobei der digitale Zwilling zumindest einen den Betriebsparameter beeinflussenden Betriebszustand der Antriebskette der Patientenliege digital abbildet und in Abhängigkeit des eingegebenen Körperwerts und des abgebildeten Betriebszustands den Betriebsparameter für das Einstellen ermittelt.

Das erfindungsgemäße Verfahren weist insbesondere folgende Vorteile auf:
Die Berücksichtigung des Körperwerts, insbesondere der Masse und/oder der Statur, des Patienten ermöglicht vorteilhafterweise ein patientenindividuelles Bewegen der Patientenliege. Das patientenindividuelle Bewegen erfolgt vorzugsweise unterschiedlich z.B. für einen kleinen, adipösen Patienten und einen großen, schlanken Patienten, wobei die beiden Patienten aufgrund der Massenträgheit die Patientenliege und deren Antriebskette üblicherweise unterschiedlich beeinflussen.

Dass das Aufschaukeln der Patientenliege zumindest teilweise unterdrückt wird, kann die Bildqualität erhöhen. Die Erhöhung des Geschwindigkeitswerts ermöglicht vorzugsweise ein schnelleres Bewegen der Patientenliege. Das schnellere Bewegen der Patientenliege bietet den Vorteil eines erhöhten Patientendurchsatzes. Der erhöhte Geschwindigkeitswert kann insbesondere bei Untersuchung von einem Notfallpatienten vorteilhaft sein.

Weiterhin kann vorteilhafterweise eine Untersuchungsdauer der medizinischen Bildgebung reduziert sein, was den Komfort des Patienten üblicherweise erhöht. Das schnellere Bewegen kann zusätzlich zu einer Erhöhung einer Bildqualität führen, beispielsweise weil ein Bildqualität-mindernder Einfluss einer Atmung und/oder einer sonstigen Bewegung des Patienten desto unwahrscheinlicher ist, je kürzer die Untersuchungsdauer ist.

Die Bildgebungsmodalität kann ein Magnetresonanztomograph, ein Computertomograph, ein Angiographie-System, eine Röntgendurchleuchtungseinrichtung, ein Positronen-Emissions-Tomograph oder eine Kombination der zuvor genannten Modalitäten sein. Die Bildgebungsmodalität ist für eine Durchführung der medizinischen Bildgebung ausgebildet. Die Bildgebungsmodalität kann zusätzlich für eine Strahlen- oder Partikeltherapie ausgebildet sein.

Jede Bildgebungsmodalität weist einen Aufnahmebereich auf, in welchem sich der Patient für die Durchführung der medizinischen Bildgebung befindet. Je nach Art der medizinischen Bildgebung und/oder der Bildgebungsmodalität variiert typischerweise eine Ausdehnung des Aufnahmebereichs und/oder die Untersuchungsdauer.

Die erfindungsgemäße Patientenliege teilt die zuvor beschriebenen Vorteile, ist zur Bewegung eines gelagerten Patienten ausgebildet und weist
- eine Liegenbrett zur Lagerung des Patienten,
- eine Antriebskette zum Bewegen der Patientenliege und
- eine Steuereinheit für die Steuerung der Antriebskette auf.

Die Lagerung des Patienten auf der Patientenliege entspricht typischerweise der Lagerung des Patienten auf dem Liegenbrett.

Das Bewegen der Patientenliege kann ein absolutes Bewegen der Patientenliege gemeinsam mit der Antriebskette umfassen, so dass beispielsweise die Patientenliege als Einheit mittels der Antriebskette im Aufnahmebereich bewegbar ist. Beispielsweise kann die Patientenliege eine verfahrbare Plattform aufweisen, auf welcher das Liegenbrett und die Antriebskette angeordnet sind. Die Antriebskette kann in diesem Fall die verfahrbare Plattform und somit die Patientenliege bewegen. Alternativ oder zusätzlich kann das Bewegen der Patientenliege ein relatives Bewegen des Liegenbretts relativ zur Antriebskette und/oder relativ zu der Plattform der Patientenliege umfassen. Die Plattform kann in diesem Fall zusätzlich selbst verfahrbar sein.

Der Patient liegt typischerweise ruhig auf der Patientenliege, während die Patientenliege bewegt wird und/oder die die medizinische Bildgebung durchgeführt wird. Eine Decke und/oder ein Gurt können die Stabilisierung des Patienten unterstützen.

Typischerweise wird der Patient in einem Abstand zur Bildgebungsmodalität außerhalb des Aufnahmebereichs auf der Patientenliege gelagert und dann für die Durchführung der medizinischen Bildgebung in den Aufnahmebereich verfahren, indem die Patientenliege mit dem auf der Patientenliege gelagerten Patienten bewegt wird. Das Bewegen der Patientenliege entspricht insbesondere einem Verfahren der Patientenliege. Die Patientenliege mit dem Patienten kann in den Aufnahmebereich auf einer Seite hinein bewegt werden und auf der anderen Seite heraus bewegt werden. Es ist denkbar, dass die Patientenliege vollständig auf der einen Seite und/oder auf der anderen Seite aus dem Aufnahmebereich heraus bewegt wird. Die Bewegung der Patientenliege kann grundsätzlich relativ zum Aufnahmebereich in alle drei Raumachsen erfolgen. Regelmäßig ist eine Seitwärtsbewegung der Patientenliege nicht vorgesehen. Die Patientenliege führt typischerweise eine Hub- und/oder Horizontalbewegung aus. Wenn die Patientenliege die Hubbewegung und die Horizontalbewegung ausführt, liegt typischerweise eine schräge Bewegung vor.

Die Antriebskette weist Antriebsmittel für ein Bewegen der Patientenliege innerhalb des Aufnahmebereichs auf. Die Antriebsmittel können einen Antriebsstrang, Räder, einen Motor und/oder ein Getriebe umfassen. Die Antriebskette kann die Patientenliege typischerweise zusätzlich außerhalb des Aufnahmebereichs bewegen. Die Patientenliege, insbesondere die Plattform der Patientenliege, steht typischerweise mittels der Räder mit dem Boden in Kontakt.

Erfindungsgemäß ist der Körperwert die Masse des Patienten oder die Statur des Patienten oder ein Tupel aus der Masse und der Statur. Im letzteren Fall sind also die Masse und die Statur Körperwerte des Patienten. Die Masse des Patienten entspricht insbesondere einem Gewicht des Patienten. Die Statur des Patienten beschreibt insbesondere eine Körperlänge des Patienten, eine Proportion des Patienten, einen Umriss des Patienten und/oder eine Positionierung des Patienten auf der Patientenliege. Die Statur entspricht insbesondere einer Masseverteilung der Masse des Patienten auf der Patientenliege.

Der Körperwert ist insbesondere ein biometrisches Merkmal des Patienten. Der Körperwert kann als Kennzahl des Patienten bezeichnet werden. Der Körperwert unterscheidet sich typischerweise von dem Patienten zu einem anderen Patienten. Das Bewegen der Patientenliege mit dem Patienten unterscheidet sich von einem Bewegen der Patientenliege mit dem anderen Patienten beispielsweise aufgrund der Massenträgheit, deren Einfluss auf das Aufschaukeln von der Masse und/oder der Statur der beiden Patienten regelmäßig abhängt.

Das Bereitstellen des Körperwerts des Patienten umfasst insbesondere das Bereitstellen der Masse und/oder der Statur. Das Bereitstellen kann ein Erfassen des Körperwerts und/oder ein Speichern des Körperwerts und/oder ein Abrufen des Körperwerts umfassen. Der Körperwert kann in einer Speichereinheit zwischengespeichert werden. Der Körperwert kann aus der Speichereinheit abgerufen werden. Das Bereitstellen des Körperwerts kann mittels einer Schnittstelle erfolgen. Das Bereitstellen des Körperwerts kann ein Übertragen des Körperwerts an die Steuereinheit der Antriebskette umfassen.

Der Körperwert kann beispielsweise vor der Lagerung oder nach der Lagerung des Patienten auf der Patientenliege mittels eines Sensors erfasst werden. Der Körperwert kann in einer Patientenakte bereitgestellt werden. Das Erfassen kann mittels einer, insbesondere stereoskopischen und/oder Wärmebild-, Kamera erfolgen. Grundsätzlich ist das Erfassen des Körperwerts mittels einer Wiegevorrichtung und/oder aus einem Betriebszustand der Patientenliege denkbar, welcher an den Patienten und dem somit zusätzlichen Körperwert angepasst wird. Der Betriebszustand kann in diesem Fall ein Motormoment der Antriebskette abbilden, welches aufgrund einer der Lagerung des Patienten zuordenbaren Reibung verändert ist, was das Erfassen des Körperwerts z.B. aus einem Körperwert-Reibung-Modell ermöglicht.

Der Betriebsparameter der Antriebskette beeinflusst den Betrieb der Antriebskette. Der Betrieb der Antriebskette variiert in Abhängigkeit einer Variation des Betriebsparameters. Der Betriebsparameter steuert insbesondere den Betrieb der Antriebskette. Der Betrieb der Antriebskette und/oder der Betriebsparameter kann zeitlich veränderbar, insbesondere dynamisch geregelt, sein.

Das Einstellen des Betriebsparameters erfolgt typischerweise in der Steuereinheit. Die Steuereinheit ist zur Steuerung der Antriebskette insbesondere durch das Einstellen des Betriebsparameters ausgebildet. Die Steuereinheit weist insbesondere Steuerprogrammcodemittel für das Einstellen des Betriebsparameters auf. Alternativ oder zusätzlich kann die Steuereinheit eine analoge Schaltung für das Einstellen des Betriebsparameters aufweisen. Die Steuereinheit kann Teil der Antriebskette oder der Patientenliege sein. Grundsätzlich ist es denkbar, dass die Steuereinheit außerhalb der Patientenliege angeordnet ist. Das Einstellen des Betriebsparameters kann über eine kabelgebundene Verbindung oder kabellos erfolgen. Die Steuereinheit kann eine Schnittstelle für das Übertragen des Betriebsparameters und/oder die Antriebskette eine Schnittstelle für das Empfangen des Betriebsparameters umfassen. Das Einstellen des Betriebsparameters kann ein Übertragen und/oder ein Antriebsketten-seitiges Empfangen umfassen.

Das Einstellen des Betriebsparameters kann mehrmals, insbesondere getaktet, vorzugsweise wiederholt erfolgen. Das Einstellen des Betriebsparameters erfolgt in Abhängigkeit des Körperwerts derart, dass der Betriebsparameter von dem Körperwert abhängt. Typischerweise hängt der Betriebsparameter zusätzlich von einem Betriebszustand der Patientenliege ab. Das Einstellen des Betriebsparameters erfolgt insbesondere in Abhängigkeit des Körperwerts und dem Betriebszustand der Patientenliege. Der Betriebszustand kann insbesondere von dem Betriebsparameter abhängen und/oder umgekehrt.

Näherungsweise wird der Betriebsparameter derart eingestellt, dass der Geschwindigkeitswert desto höher ist, je kleiner und/oder schmächtiger der Patient ist. Der Geschwindigkeitswert umfasst insbesondere die Geschwindigkeit und/oder die Beschleunigung. Vorteilhafterweise wird der Betriebsparameter in Abhängigkeit des Körperwerts derart eingestellt, dass der Geschwindigkeitswert bis zu einem Maximalwert erhöht wird. Der Maximalwert des Geschwindigkeitswerts kann durch eine Leistungsgrenze der Antriebskette vorgegeben sein. Der Maximalwert des Geschwindigkeitswerts kann alternativ einem Schwellwert entsprechen, welcher Körperwert-abhängig ist, um ein Verrutschen und/oder Abheben des Patienten zu verhindern. In einem anderen Fall kann der Maximalwert des Geschwindigkeitswerts durch einen Nutzer der Patientenliege vorgegeben werden. Insbesondere die Steuereinheit ist dazu eingerichtet, den Betriebsparameter derart einzustellen, dass der Geschwindigkeitswert nur bis zum Maximalwert erhöht wird. Grundsätzlich ist es denkbar, dass bei der Erhöhung des Geschwindigkeitswerts der Maximalwert insbesondere aus Komfort- und/oder Verschleiß- und/oder Sicherheitsgründen (nur) zu 50%, beispielsweise 80% oder 90% ausgereizt wird, so dass eine Leistungsreserve bis zum Maximalwert erhalten bleibt. Bei der Hubbewegung kann der Maximalwert beispielsweise mehr als 10 mm/s und/oder weniger als 100 mm/s, insbesondere 50 mm/s betragen. Bei der Horizontalbewegung kann der Maximalwert beispielsweise mehr als 50 mm/s und/oder weniger als 1000 mm/s, insbesondere 500 mm/s betragen.

Ein nicht unterdrücktes Schaukeln kann sich in einem sogenannten Wabbeln des Patienten, insbesondere von Weichteilen des Patienten, zeigen. Das Unterdrücken des Aufschaukelns der Patientenliege umfasst das Unterdrücken des Aufschaukelns des auf der Patientenliege gelagerten Patienten. Das Unterdrücken des Aufschaukelns entspricht insbesondere einem Verhindern, dass die Patientenliege zunehmend in Schwingung gerät. Das Unterdrücken des Aufschaukelns umfasst insbesondere ein Dämpfen der Schwingung. Das Unterdrücken des Aufschaukelns erfolgt insbesondere nur teilweise und schließt somit eine gewisse Restschwingung nicht vollständig aus. Die Restschwingung kann beispielsweise unterhalb eines Schwellwerts relativ zu einem nicht unterdrückten Schaukeln liegen.

Das Unterdrücken des Aufschaukelns erfordert typischerweise eine ganzheitliche, sprich systemische Betrachtung der Patientenliege und des auf der Patientenliege gelagerten Patienten, weil offensichtlich die Patientenliege und der Patient mittels der Antriebskette gemeinsam bewegt werden. Für das Unterdrücken des Aufschaukelns wird insbesondere der Körperwert und der Betriebszustand der Patientenliege berücksichtigt.

Das Einstellen des Betriebsparameters umfasst insbesondere ein erneutes Anpassen des Betriebsparameters und/oder ein Regeln der Antriebskette bei der Bewegung der Patientenliege, so dass bei der Bewegung der Patientenliege das Aufschaukeln zumindest teilweise unterdrückt wird. Das Regeln der Antriebskette umfasst üblicherweise ein Überwachen und/oder Erfassen des Betriebszustands und/oder eines tatsächlichen gemäß dem erhöhten Geschwindigkeitswert vorgegebenen Geschwindigkeitswerts und/oder der Einhaltung des eingestellten Betriebsparameters. In einem Fall, wenn ein eingestellter Betriebsparameter das Aufschaukeln der Patientenliege bei der Bewegung der Patientenliege nicht zumindest teilweise unterdrückt, kann die Steuereinheit den Betriebsparameter erneut einstellen. Dafür kann eine Anpassung des Betriebszustands nötig sein. Das Einstellen des Betriebsparameters kann laufend während des Bewegens der Patientenliege wiederholt erfolgen. Bei der Bewegung der Patientenliege umfasst insbesondere den Zeitraum nach einem initialen Stillstand der Patientenliege und vor einem darauffolgenden Stillstand der Patientenliege, währenddessen die Patientenliege bewegt wird. Bei der Bewegung der Patientenliege umfasst alternativ oder zusätzlich den Zeitraum der Untersuchungsdauer.

Die Patientenliege wird mittels der Antriebskette derart bewegt, dass die medizinische Bildgebung des Patienten durchgeführt werden kann. Die Patientenliege wird dabei insbesondere in Richtung des Aufnahmebereichs und/oder in den Aufnahmebereich hinein und/oder durch den Aufnahmebereich hindurch bewegt. Die Bewegung der Patientenliege erfolgt gemäß dem erhöhten Geschwindigkeitswert. Bei der Bewegung der Patientenliege kann die Steuereinheit die Antriebskette vorzugsweise regeln und/oder den Betriebsparameter einstellen.

Eine Ausführungsform sieht vor, dass vor dem Einstellen des Betriebsparameters zumindest ein Sequenzparameter eines Bildgebungsprotokolls, gemäß welchem die medizinische Bildgebung durchgeführt wird, abgerufen wird und wobei der Betriebsparameter gemäß dem zumindest einen Sequenzparameter eingestellt wird. Diese Ausführungsform ist insbesondere vorteilhaft, weil die Art der medizinischen Bildgebung beim Bewegen der Patientenliege berücksichtigt werden kann. Beispielsweise kann ein bestimmtes Bildgebungsprotokoll, welches die Bildgebung einer Physiologie des Patienten und/oder eine Kontrastmittelanreicherung im Patienten vorsieht, überhaupt oder mit einer verbesserten Bildqualität durchgeführt werden. Die Berücksichtigung des Sequenzparameters beim Einstellen des Betriebsparameters bei gleichzeitiger Unterdrückung des Aufschaukelns ermöglicht vorteilhafterweise ein Bildgebungsprotokoll- und Patientenabhängiges Bewegen der Patientenliege. Wenn der Betriebsparameter gemäß dem zumindest einen Sequenzparameter eingestellt wird, kann der Geschwindigkeitswert in Abhängigkeit von dem Sequenzparameter stärker erhöht werden, beispielsweise durch ein Einsetzen der Leistungsreserve. Der zumindest eine Sequenzparameter kann beispielsweise von der Bildgebungsmodalität für das Abrufen bereitgestellt, insbesondere übertragen werden. Das Abrufen kann beispielsweise durch die Steuereinheit erfolgen. Die Steuereinheit kann eine Schnittstelle für das Abrufen des zumindest einen Sequenzparameters aufweisen.

Eine Ausführungsform sieht vor, dass der zumindest eine Sequenzparameter ein Bewegen der Patientenliege innerhalb des Aufnahmebereichs vorsieht. Diese Ausführungsform ist insbesondere vorteilhaft, weil dadurch die gemäß dem zumindest einen Sequenzparameter vorgesehene Bewegung der Patientenliege beim Einstellen des Betriebsparameters berücksichtigt werden kann. Die Berücksichtigung kann in Form einer relativen Verringerung oder Verstärkung der Erhöhung des Geschwindigkeitswert erfolgen.

Eine Ausführungsform sieht vor, dass die vorgesehene Bewegung zumindest eine Richtungsänderung umfasst. Definiert ist eine Richtungsänderung, dass sich eine gerichtete Bewegungsachse, entlang welcher die Patientenliege bewegt wird, verändert. Beispielsweise liegt eine Richtungsänderung vor, wenn die Patientenliege eine Hubbewegung und darauffolgend eine Horizontalbewegung ausführt oder umgekehrt. Bei einer Richtungsänderung ist insbesondere der Einfluss der Massenträgheit auf das Aufschaukeln besonders signifikant, so dass sich das Aufschaukeln verschiedener Patienten unterscheiden kann. Das nicht unterdrückte Aufschaukeln ist typischerweise desto wahrscheinlicher, je größer die Richtungsänderung ist. Diese Ausführungsform bietet insbesondere den Vorteil, dass dieser für die zumindest eine Richtungsänderung wesentliche Einfluss beim Einstellen des Betriebsparameters berücksichtigt werden kann.

Eine Ausführungsform sieht vor, dass die zumindest eine Richtungsänderung 180° beträgt. Eine Richtungsänderung um 180° wird beispielsweise Zick-Zack genannt. Eine derartige Richtungsänderung führt typischerweise dazu, dass die Patientenliege hin und her, insbesondere zunächst entlang der gerichteten Bewegungsachse und daraufhin entlang einer zur gerichteten Bewegungsachse antiparallelen Bewegungsachse bewegt wird. Zumindest ein Bildgebungsprotokoll sieht eine derartige Richtungsänderung vor, um beispielsweise einen zu untersuchenden Bereich, der größer ist als der Aufnahmebereich, des Patienten vorzugsweise mehrmals zu untersuchen. Regelmäßig betragen mehrere Richtungsänderungen beim Bewegen der Patientenliege 180°. Es ist besonders vorteilhaft, wenn der Betriebsparameter gemäß der zumindest einen Richtungsänderung eingestellt wird und das Aufschaukeln zumindest teilweise unterdrückt wird.

Eine Ausführungsform sieht vor, dass das Einstellen des Betriebsparameters ein Auswählen einer Motorstromkennlinie der Antriebskette in Abhängigkeit des Körperwerts umfasst. Typischerweise korreliert der durch die Motorstromkennlinie vorgegebene Motorstrom, welcher in der Antriebskette verbraucht wird, mit dem Geschwindigkeitswert. Die Motorstromkennlinie korreliert insbesondere mit einem von der Antriebskette für das Bewegen der Patientenliege bereitgestellte Motormoment. Die Motorstromkennlinie kann beispielsweise mit weiteren Motorstromkennlinien für das Auswählen in einer Speichereinheit bereitgestellt werden. Die Steuereinheit wählt typischerweise die Motorstromkennlinie aus, insbesondere in Abhängigkeit vom Körperwert und der Patientenliege.

Eine Ausführungsform sieht vor, dass der Geschwindigkeitswert eine Beschleunigungsrampe der Patientenliege und/oder eine maximale Geschwindigkeit der Patientenliege vorgibt. Die Beschleunigungsrampe kann ein Erhöhen der Geschwindigkeit und/oder ein Verringern der Geschwindigkeit für ein Abbremsen der Patientenliege vorsehen. Die Beschleunigungsrampe hängt insbesondere vom der Beschleunigung ab. Die maximale Geschwindigkeit der Patientenliege korreliert typischerweise mit dem Maximalwert des Geschwindigkeitswerts. Die Betriebsparameter kann beispielsweise zunächst die Beschleunigungsrampe und daraufhin die maximale Geschwindigkeit der Patientenliege einstellen.

Eine Ausführungsform sieht vor, dass der dem einzustellende Betriebsparameter entsprechende erhöhte Geschwindigkeitswert auf einer Anzeigeeinheit für einen Nutzer der Patientenliege bereitgestellt wird. Die Anzeigeeinheit kann beispielsweise einen Bildschirm umfassen, insbesondere als Teil eines Smartphones oder eines Tablets ausgeführt sein. Diese Ausführungsform ist insbesondere vorteilhaft, weil der Körperwert-abhängige Geschwindigkeitswert dem Nutzer angezeigt wird. Der Nutzer kann den einzustellenden Betriebsparameter insbesondere für das Bewegen der Patientenliege freigeben. Grundsätzlich ist es denkbar, dass der Nutzer mittels einer Schaltereinheit den einzustellenden Betriebsparameter anpassen kann, wenn der erhöhte Geschwindigkeitswert vor dem Bewegen der Patientenliege dem Nutzer für eine Freigabe angezeigt wird. Alternativ oder zusätzlich kann der Nutzer den Maximalwert des Geschwindigkeitswerts beispielsweise mittels der Schaltereinheit anpassen, wenn der einzustellende Betriebsparameter den zuvor eingestellten Maximalwert übersteigt. Die Schaltereinheit kann einen Drehknopf für eine stufenlose Einstellung umfassen.

Eine Ausführungsform sieht vor, dass der Körperwert vor der Lagerung des Patienten auf der Patientenliege erfasst wird. Diese Ausführungsform ist insbesondere vorteilhaft, weil bereits vor der Lagerung des Patienten auf der Patientenliege der Betriebsparameter eingestellt werden kann.

Erfindungsgemäß wird der Körperwert in einen digitalen Zwilling der Antriebskette der Patientenliege eingegeben, wobei der digitale Zwilling zumindest einen den Betriebsparameter beeinflussenden Betriebszustand der Antriebskette der Patientenliege digital abbildet und in Abhängigkeit des eingegebenen Körperwerts und des abgebildeten Betriebszustands den Betriebsparameter für das Einstellen ermittelt. Der digitale Zwilling kann zusätzlich die Patientenliege und/oder den Patienten digital abbilden. Dies ist als eine Möglichkeit der ganzheitlichen, sprich systemischen Betrachtung der Patientenliege und des auf der Patientenliege gelagerten Patienten besonders vorteilhaft. Der digitale Zwilling bietet insbesondere den Vorteil, dass dieser typischerweise das Bewegen der Patientenliege wenigstens näherungsweise simulieren und/oder modellieren kann. Der digitale Zwilling bildet in der sowie auf die Antriebskette und/oder Patientenliege wirkende Kräfte, Motormomente, Belastungsfälle, Verformungen und/oder Reibungen wenigstens teilweise, vorzugsweise vollständig, in dem Betriebszustand ab. Das Eingeben des Körperwerts kann durch die Steuereinheit erfolgen. Grundsätzlich ist es denkbar, dass der digitale Zwilling eine Schnittstelle für das Empfangen des Körperwerts umfasst. Der digitale Zwilling ist typischerweise in Programmcodemitteln abgebildet, welche in einer Recheneinheit ausführbar sind. Die Recheneinheit ist Teil der Steuereinheit. Das Ermitteln des Betriebsparameters kann das Simulieren und/oder das Modellieren des Bewegens der Patientenliege umfassen, wobei ein Ausgangsparameter des Simulierens und/oder des Modellierens der für das Einstellen vorgesehene Betriebsparameter ist. In den Betriebszustand fließen insbesondere der Körperwert, die Masse und/oder die Statur, des Patienten, der Zustand der Antriebskette und/oder der Zustand der Patientenliege ein. Der Zustand der Antriebskette und/oder der Zustand der Patientenliege kann eine Funktion der jeweiligen Elemente, z.B. der Antriebsmittel, und/oder deren Betriebszeiten und/oder deren Wartungsintervalle und/oder deren Verformungen aufgrund des gelagerten Patienten abbilden.

Eine Ausführungsform sieht vor, dass vor dem Ermitteln des Betriebsparameters der zumindest eine den Betriebsparameter beeinflussende Betriebszustand an den Körperwert angepasst wird. Diese Ausführungsform ist insbesondere vorteilhaft, weil das Ermitteln des Betriebsparameter prospektiv erfolgen kann.

Eine Ausführungsform sieht vor, dass nach dem Bewegen der Patientenliege der Betriebszustand an den durch das Bewegen der Patientenliege erfahrenen Verschleiß derart angepasst wird, dass ein Reibungswert erhöht wird. Diese Ausführungsform bietet insbesondere den Vorteil, dass der digitale Zwilling aufgrund der Bewegung der Patientenliege aktualisiert wird und somit ein erneutes Einstellen eines Betriebsparameters für ein weiteres Bewegen der Patientenliege präziser sein kann. Der Verschleiß kann beispielsweise durch das Simulieren und/oder Modellieren des Bewegens der Patientenliege ermittelt und/oder mittels eines Verschleißsensors erfasst werden. Üblicherweise entsteht durch das Bewegen der Patientenliege der Verschleiß, welcher typischerweise zu einer höheren Reibung führt, was in dem Reibungswert abgebildet wird.

Eine Ausführungsform sieht vor, dass der digitale Zwilling in einer Cloud-Computer-Einrichtung betrieben wird. Der Zugriff des digitalen Zwillings auf die Schnittstelle der Steuereinheit für das Übertragen des ermittelten Betriebsparameter und/oder der Zugriff der Steuereinheit auf den digitalen Zwilling zur Eingabe des Körperwerts erfolgt typischerweise mittels eines Computernetzwerks. Die Cloud-Computer-Einrichtung kann in einem Cloud-Server betrieben werden. Grundsätzlich ist es denkbar, dass ein Teil des Cloud-Servers als Steuereinheit ausgeführt ist.

Das erfindungsgemäße Computerprogrammprodukt, welches direkt in einen Speicher einer Recheneinheit einer Steuereinheit einer Patientenliege ladbar ist, weist Programmcodemittel auf, um das Verfahren auszuführen, wenn das Computerprogrammprodukt in der Recheneinheit ausgeführt wird.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network-(WAN) bzw. einer (Wireless-)Local-Area-Network-Verbindung (WLAN oder LAN) mit der Recheneinheit und/oder Steuereinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Verfahren für ein Bewegen einer Patientenliege in Abhängigkeit eines Körperwerts eines auf der Patientenliege für eine medizinische Bildgebung gelagerten Patienten,
Fig. 2 ein erstes Ausführungsbeispiel des Verfahrens,
Fig. 3 ein zweites Ausführungsbeispiel des Verfahrens,
Fig. 4 ein drittes Ausführungsbeispiel des Verfahrens,
Fig. 5 ein viertes Ausführungsbeispiel des Verfahrens,
Fig. 6 ein fünftes Ausführungsbeispiel des Verfahrens,
Fig. 7 ein sechstes Ausführungsbeispiel des Verfahrens,
Fig. 8 ein siebtes Ausführungsbeispiel des Verfahrens und
Fig. 9 eine erfindungsgemäße Patientenliege.

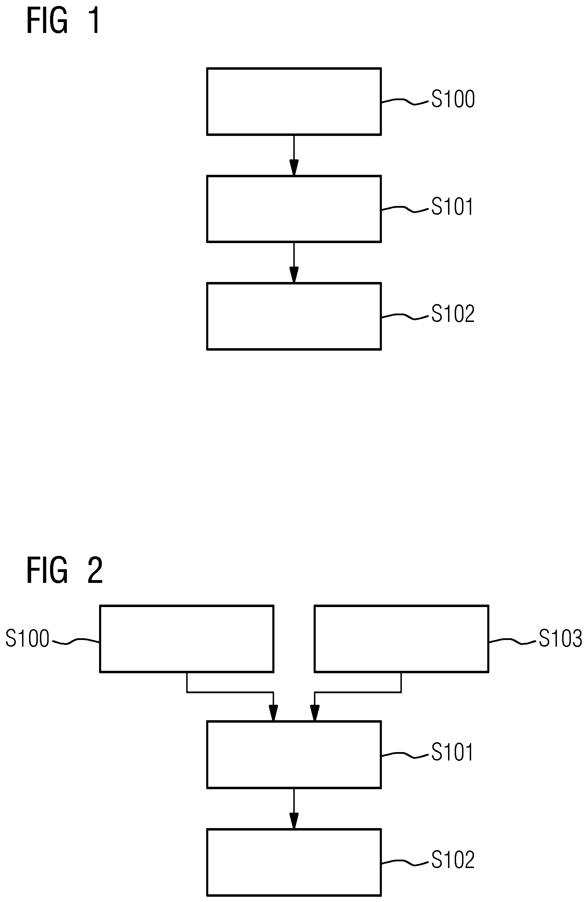

**Fig. 1** zeigt ein Verfahren für ein Bewegen einer Patientenliege in Abhängigkeit eines Körperwerts eines auf der Patientenliege für eine medizinische Bildgebung gelagerten Patienten, wobei die medizinische Bildgebung in einer Bildgebungsmodalität mit einem Aufnahmebereich erfolgt, wobei die Patientenliege innerhalb des Aufnahmebereichs mittels einer Antriebskette bewegbar ist, in einem Flussdiagramm mit den Schritten S100 bis S102:
Verfahrensschritt S100 kennzeichnet ein Bereitstellen eines Körperwerts, insbesondere einer Masse und/oder einer Statur, des Patienten.

Verfahrensschritt S101 kennzeichnet ein Einstellen eines Betriebsparameters der Antriebskette mittels einer Steuereinheit für die Steuerung der Antriebskette in Abhängigkeit des Körperwerts derart, dass ein Geschwindigkeitswert der Patientenliege erhöht wird und ein Aufschaukeln der Patientenliege bei der Bewegung der Patientenliege zumindest teilweise unterdrückt wird.

Verfahrensschritt S102 kennzeichnet ein Bewegen der Patientenliege mittels der Antriebskette gemäß dem erhöhten Geschwindigkeitswert zur Durchführung der medizinischen Bildgebung.

**Fig. 2** zeigt ein erstes Ausführungsbeispiel des in Fig. 1 gezeigten Verfahrens in einem Flussdiagramm:
Verfahrensschritt S103 kennzeichnet, dass vor dem Einstellen des Betriebsparameters zumindest ein Sequenzparameter eines Bildgebungsprotokolls, gemäß welchem die medizinische Bildgebung durchgeführt wird, abgerufen wird und wobei der Betriebsparameter gemäß dem zumindest einen Sequenzparameter eingestellt wird.

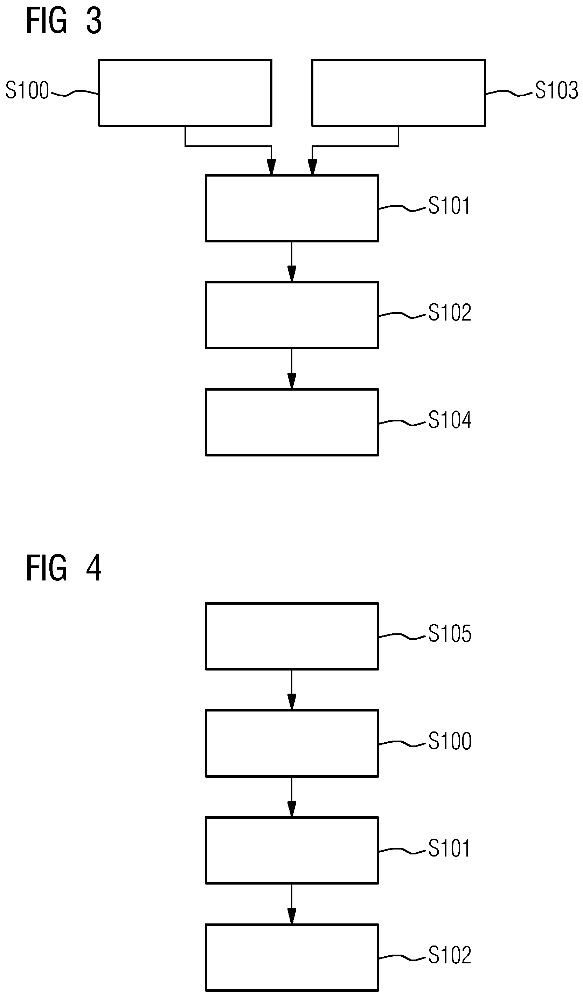

**Fig. 3** zeigt ein zweites Ausführungsbeispiel des in Fig. 2 gezeigten Verfahrens in einem Flussdiagramm:
Verfahrensschritt S104 kennzeichnet, dass der zumindest eine Sequenzparameter ein Bewegen der Patientenliege innerhalb des Aufnahmebereichs vorsieht.

Diese Ausführungsform kann insbesondere dadurch weitergebildet werden, dass die vorgesehene Bewegung zumindest eine Richtungsänderung umfasst, insbesondere wobei die zumindest eine Richtungsänderung 180° beträgt.

**Fig. 4** zeigt ein drittes Ausführungsbeispiel des in Fig. 1 gezeigten Verfahrens in einem Flussdiagramm:
Verfahrensschritt S105 kennzeichnet, dass der Körperwert vor der Lagerung des Patienten auf der Patientenliege erfasst wird.

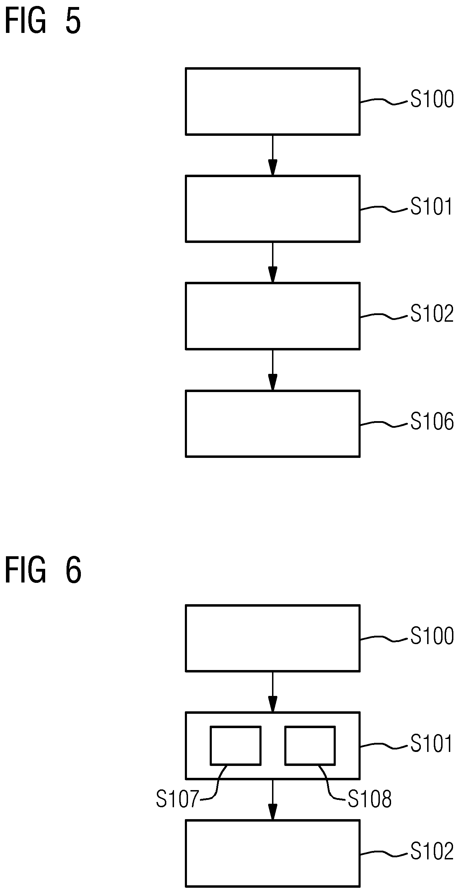

**Fig. 5** zeigt ein viertes Ausführungsbeispiel des in Fig. 1 gezeigten Verfahrens in einem Flussdiagramm:
Verfahrensschritt S106 kennzeichnet, dass der dem einzustellende Betriebsparameter entsprechende erhöhte Geschwindigkeitswert auf einer Anzeigeeinheit für einen Nutzer der Patientenliege bereitgestellt wird.

**Fig. 6** zeigt ein fünftes Ausführungsbeispiel des in Fig. 1 gezeigten Verfahrens in einem Flussdiagramm:
Verfahrensschritt S107 kennzeichnet, dass das Einstellen des Betriebsparameters ein Auswählen einer Motorstromkennlinie der Antriebskette in Abhängigkeit des Körperwerts umfasst.

Der alternative oder zusätzliche Verfahrensschritt S108 kennzeichnet, dass der Geschwindigkeitswert eine Beschleunigungsrampe der Patientenliege und/oder eine maximale Geschwindigkeit der Patientenliege vorgibt.

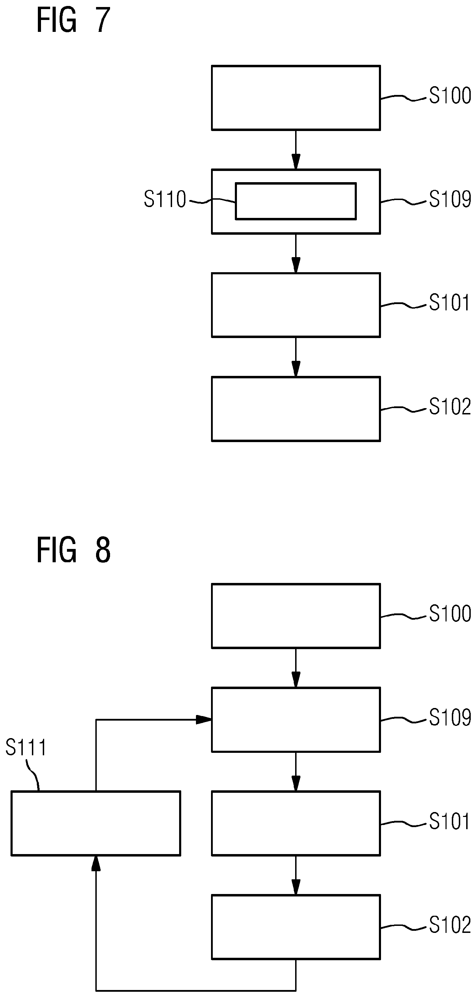

**Fig. 7** zeigt ein sechstes Ausführungsbeispiel des in Fig. 1 gezeigten Verfahrens in einem Flussdiagramm: Verfahrensschritt S109 kennzeichnet, dass der Körperwert in
einen digitalen Zwilling der Antriebskette der Patientenliege eingegeben wird, wobei der digitale Zwilling zumindest einen den Betriebsparameter beeinflussenden Betriebszustand der Antriebskette der Patientenliege digital abbildet und in Abhängigkeit des eingegebenen Körperwerts und des abgebildeten Betriebszustands den Betriebsparameter für das Einstellen ermittelt.

Ein optionaler Verfahrensschritt S110 kennzeichnet, dass vor dem Ermitteln des Betriebsparameters der zumindest eine den Betriebsparameter beeinflussende Betriebszustand an den Körperwert angepasst wird.

Grundsätzlich ist es denkbar, dass der digitale Zwilling in einer Cloud-Computer-Einrichtung betrieben wird.

**Fig. 8** zeigt ein siebtes Ausführungsbeispiel des in Fig. 7 gezeigten Verfahrens in einem Flussdiagramm:
Verfahrensschritt S111 kennzeichnet, dass nach dem Bewegen der Patientenliege der Betriebszustand an den durch das Bewegen der Patientenliege erfahrenen Verschleiß derart angepasst wird, dass ein Reibungswert erhöht wird.

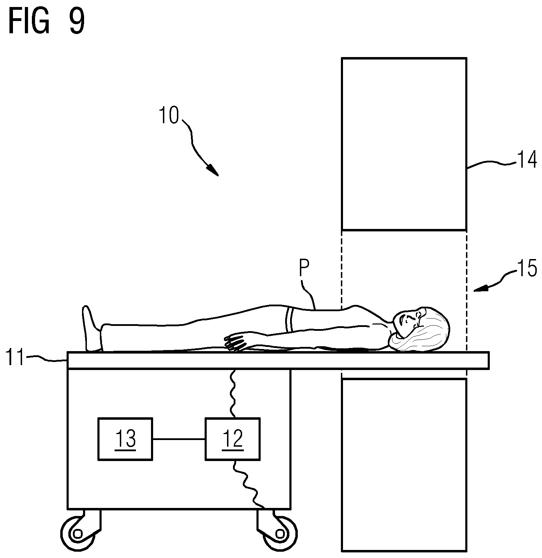

**Fig. 9** zeigt die erfindungsgemäße Patientenliege 10. Die Patientenliege 10 ist zur Bewegung eines auf ihr gelagerten Patienten P ausgebildet. Die Patientenliege 10 weist ein Liegenbrett 11, eine Antriebskette 12 und eine Steuereinheit 13 auf.

Das Liegenbrett 11 ist zur Lagerung des Patienten P ausgebildet. Das Liegenbrett ist typischerweise flach und länglich. Das Liegenbrett 11 ist insbesondere relativ zu einem Boden, auf dem die Patientenliege 10 steht, horizontal und/oder vertikal bewegbar.

Die Antriebskette 12 ist zum Bewegen der Patientenliege 10 ausgebildet. Die Antriebskette 12 kann die Patientenliege 10 insbesondere absolut, also gemeinsam mit der Antriebskette 12 relativ zu einer Position auf dem Boden, und/oder relativ, also insbesondere das Liegenbrett 11 relativ zur Antriebskette 12, bewegen.

Die Steuereinheit 13 ist für die Steuerung der Antriebskette 12 ausgebildet. Die Steuereinheit ist dafür insbesondere mit der Antriebskette 12 und/oder der Patientenliege 10 und/oder dem Liegenbrett 11 verbunden. In diesem Ausführungsbeispiel ist die Steuereinheit 13 als Teil der Patientenliege 10 ausgeführt, so dass sie 13 auf der Plattform der Patientenliege 10 gemeinsam mit der Antriebskette 12 angeordnet ist.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Bewegen einer Patientenliege in Abhängigkeit eines Körperwerts eines auf der Patientenliege für eine medizinische Bildgebung gelagerten Patienten, wobei die medizinische Bildgebung in einer Bildgebungsmodalität mit einem Aufnahmebereich erfolgt, wobei die Patientenliege innerhalb des Aufnahmebereichs mittels einer Antriebskette bewegbar ist, mit den folgenden Schritten:
- Bereitstellen eines Körperwerts, insbesondere einer Masse und/oder einer Statur, des Patienten,
- Einstellen eines Betriebsparameters der Antriebskette mittels einer Steuereinheit für die Steuerung der Antriebskette in Abhängigkeit des Körperwerts derart, dass ein Geschwindigkeitswert der Patientenliege bis zu einem vorgegebenen oder Körperwert-abhängigen Maximalwert erhöht wird, und gegebenenfalls erneutes Einstellen des Betriebsparameters, damit ein Aufschaukeln der Patientenliege bei der Bewegung der Patientenliege zumindest teilweise unterdrückt wird, und
- Bewegen der Patientenliege mittels der Antriebskette gemäß dem erhöhten Geschwindigkeitswert zur Durchführung der medizinischen Bildgebung,
**dadurch gekennzeichnet, dass** der Körperwert in einen digitalen Zwilling der Antriebskette der Patientenliege eingegeben wird, wobei der digitale Zwilling zumindest einen den Betriebsparameter beeinflussenden Betriebszustand der Antriebskette der Patientenliege digital abbildet und in Abhängigkeit des eingegebenen Körperwerts und des abgebildeten Betriebszustands den Betriebsparameter für das Einstellen ermittelt.

2. Verfahren nach Anspruch 1, wobei vor dem Einstellen des Betriebsparameters zumindest ein Sequenzparameter eines Bildgebungsprotokolls, gemäß welchem die medizinische Bildgebung durchgeführt wird, abgerufen wird und wobei der Betriebsparameter gemäß dem zumindest einen Sequenzparameter eingestellt wird.

3. Verfahren nach Anspruch 2, wobei der zumindest eine Sequenzparameter ein Bewegen der Patientenliege innerhalb des Aufnahmebereichs vorsieht.

4. Verfahren nach Anspruch 3, wobei die vorgesehene Bewegung zumindest eine Richtungsänderung umfasst.

5. Verfahren nach Anspruch 4, wobei die zumindest eine Richtungsänderung 180° beträgt.

6. Verfahren nach einem der vorhergehenden Ansprüche, wobei das Einstellen des Betriebsparameters ein Auswählen einer Motorstromkennlinie der Antriebskette in Abhängigkeit des Körperwerts umfasst.

7. Verfahren nach einem der vorhergehenden Ansprüche, wobei der Geschwindigkeitswert eine Beschleunigungsrampe der Patientenliege und/oder eine maximale Geschwindigkeit der Patientenliege vorgibt.

8. Verfahren nach einem der vorhergehenden Ansprüche, wobei vor dem Ermitteln des Betriebsparameters der zumindest eine den Betriebsparameter beeinflussende Betriebszustand an den Körperwert angepasst wird.

9. Verfahren nach einem der vorhergehenden Ansprüche, wobei der digitale Zwilling in einer Cloud-Computer-Einrichtung betrieben wird.

10. Verfahren nach einem der vorhergehenden Ansprüche, wobei nach dem Bewegen der Patientenliege der Betriebszustand an den durch das Bewegen der Patientenliege erfahrenen Verschleiß derart angepasst wird, dass ein Reibungswert erhöht wird.

11. Verfahren nach einem der vorhergehenden Ansprüche, wobei der dem einzustellende Betriebsparameter entsprechende erhöhte Geschwindigkeitswert auf einer Anzeigeeinheit für einen Nutzer der Patientenliege bereitgestellt wird.

12. Verfahren nach einem der vorhergehenden Ansprüche, wobei der Körperwert vor der Lagerung des Patienten auf der Patientenliege erfasst wird.

13. Patientenliege (10) zur Bewegung eines gelagerten Patienten (P), aufweisend
- ein Liegenbrett (11) zur Lagerung des Patienten (P),
- eine Antriebskette (12) zum Bewegen der Patientenliege (10) und
- eine Steuereinheit (13) für die Steuerung der Antriebskette (12), wobei die Patientenliege (10) zur Durchführung eines Verfahrens nach einem der vorhergehenden Ansprüche ausgebildet ist.

14. Computerprogrammprodukt, welches direkt in einen Speicher einer Recheneinheit einer Steuereinheit einer Patientenliege ladbar ist, mit Programmcodemitteln, um ein Verfahren nach einem der Ansprüche 1 bis 12 auszuführen, wenn das Computerprogrammprodukt in der Recheneinheit ausgeführt wird.

## Claims

1. Method for moving a patient couch as a function of a body value of a patient positioned on the patient couch for medical imaging, wherein the medical imaging takes place in an imaging modality with a recording region, wherein the patient couch can be moved within the recording region by means of a drive chain, with the following steps:
- providing a body value, in particular a mass and/or physique, of the patient,
- setting an operating parameter of the drive chain by means of a control unit for controlling the drive chain as a function of the body value in such a manner that a speed value of the patient couch is increased up to a predefined or body value-dependent maximum value, and possibly repeatedly setting the operating parameter so that a rocking of the patient couch during the movement of the patient couch is at least partially suppressed, and
- moving the patient couch by means of the drive chain in accordance with the increased speed value to perform the medical imaging,
**characterised in that** the body value is entered into a digital twin of the drive chain of the patient couch, wherein the digital twin digitally maps at least one operating state of the drive chain of the patient couch which influences the operating parameter and, as a function of the entered body value and the mapped operating state, ascertains the operating parameter for the setting.

2. Method according to claim 1, wherein, before the setting of the operating parameter, at least one sequence parameter of an imaging protocol, in accordance with which the medical imaging is performed, is retrieved and wherein the operating parameter is set in accordance with the at least one sequence parameter.

3. Method according to claim 2, wherein the at least one sequence parameter provides a movement of the patient couch within the recording region.

4. Method according to claim 3, wherein the provided movement comprises at least one change in direction.

5. Method according to claim 4, wherein the at least one change in direction amounts to 180°.

6. Method according to one of the preceding claims, wherein the setting of the operating parameter comprises selecting a motor current characteristic curve of the drive chain as a function of the body value.

7. Method according to one of the preceding claims, wherein the speed value predefines an acceleration ramp of the patient couch and/or a maximum speed of the patient couch.

8. Method according to one of the preceding claims, wherein, before the ascertaining of the operating parameter, the at least one operating state influencing the operating parameter is adapted to the body value.

9. Method according to one of the preceding claims, wherein the digital twin is operated in a cloud computing facility.

10. Method according to one of the preceding claims, wherein, after the movement of the patient couch, the operating state is adapted to the wear experienced due to the movement of the patient couch in such a manner that a friction value is increased.

11. Method according to one of the preceding claims, wherein the increased speed value corresponding to the operating parameter to be set is provided on a display unit for a user of the patient couch.

12. Method according to one of the preceding claims, wherein the body value is detected before the patient is positioned on the patient couch.

13. Patient couch (10) for moving a positioned patient (P), having
- a couch board (11) for positioning of the patient (P),
- a drive chain (12) for moving the patient couch (10) and
- a control unit (13) for controlling the drive chain (12), wherein the patient couch (10) is embodied for performing a method according to one of the preceding claims.

14. Computer program product which can be loaded directly into a memory of a computing unit of a control unit of a patient couch, with program code means in order to carry out a method according to one of claims 1 to 12 when the computer program product is executed in the computing unit.

## Revendications

1. Procédé de déplacement d'une couchette de patient en fonction d'une valeur du corps d'un patient placé sur la couchette de patient pour une imagerie médicale, dans lequel l'imagerie médicale s'effectue suivant une modalité d'imagerie ayant une zone d'enregistrement, dans lequel la couchette de patient est mobile, au moyen d'une chaîne d'entraînement, dans la zone d'enregistrement, comprenant les stades suivants :
- se procurer une valeur du corps, en particulier une masse et/ou une stature du patient,
- régler un paramètre de fonctionnement de la chaîne d'entraînement, au moyen d'une unité de commande, pour la commande de la chaîne d'entraînement en fonction de la valeur du corps, de manière à augmenter une valeur de vitesse de la couchette du patient jusqu'à une valeur maximum donnée à l'avance ou qui dépend de la valeur du corps, et le cas échéant renouveler le réglage du paramètre de fonctionnement, afin de supprimer au moins en partie un balancement de la couchette du patient, lors du déplacement de la couchette du patient, et
- déplacer la couchette de patient au moyen de la chaîne d'entraînement en fonction de la valeur de vitesse augmentée pour effectuer l'imagerie médicale,
**caractérisé en ce que** la valeur du corps est entrée dans un double numérique de la chaîne d'entraînement de la couchette de patient, dans lequel le double numérique reproduit numériquement au moins un état de fonctionnement, influençant le paramètre de fonctionnement de la chaîne d'entraînement, de la couchette de patient et détermine, pour le réglage, le paramètre de fonctionnement en fonction de la valeur du corps entrée et de l'état de fonctionnement reproduit.

2. Procédé suivant la revendication 1, dans lequel, avant le réglage du paramètre de fonctionnement, on appelle au moins un paramètre de séquence d'un protocole d'imagerie, suivant lequel l'imagerie médicale est effectuée, et dans lequel on règle le paramètre de fonctionnement suivant le au moins un paramètre de séquence.

3. Procédé suivant la revendication 2, dans lequel le au moins un paramètre de séquence prévoit un mouvement de la couchette du patient dans la zone d'enregistrement.

4. Procédé suivant la revendication 3, dans lequel le mouvement prévu comprend au moins un changement de direction.

5. Procédé suivant la revendication 4, dans lequel le au moins un changement de direction s'élève à 180°.

6. Procédé suivant l'une des revendications précédentes, dans lequel le réglage du paramètre de fonctionnement comprend une sélection d'une courbe caractéristique de moteur de la chaîne d'entraînement en fonction de la valeur du corps.

7. Procédé suivant l'une des revendications précédentes, dans lequel la valeur de vitesse prescrit une rampe d'accélération de la couchette du patient et/ou une vitesse maximum de la couchette du patient.

8. Procédé suivant l'une des revendications précédentes, dans lequel, avant la détermination du paramètre de fonctionnement, on adapte à la valeur du corps le au moins un état de fonctionnement influençant le paramètre de fonctionnement.

9. Procédé suivant l'une des revendications précédentes, dans lequel on fait fonctionner le double numérique dans un dispositif informatique à nuage.

10. Procédé suivant l'une des revendications précédentes, dans lequel, après le déplacement de la couchette de patient, on adapte l'état de fonctionnement à l'usure subie par le mouvement de la couchette de patient, de manière à augmenter une valeur de frottement.

11. Procédé suivant l'une des revendications précédentes, dans lequel on donne, à un utilisateur de la couchette de patient sur une unité d'affichage, la valeur de vitesse augmentée conformément au paramètre de fonctionnement à régler.

12. Procédé suivant l'une des revendications précédentes, dans lequel on détecte la valeur du corps avant de mettre le patient sur la couchette de patient.

13. Couchette (10) de patient pour le déplacement d'un patient (P) mis en place, comportant
- une table (11) de couchette pour la mise en place du patient (P),
- une chaîne (12) d'entraînement pour le déplacement de la couchette (10) de patient et
- une unité (13) de commande pour la commande de la chaîne (12) d'entraînement, dans lequel la couchette (10) de patient est constituée pour effectuer un procédé suivant l'une des revendications précédentes.

14. Produit de programme d'ordinateur, qui peut être chargé directement dans la mémoire d'une unité informatique d'une unité de commande d'une couchette de patient, comprenant des moyens de code de programme, pour exécuter un procédé suivant l'une des revendications 1 à 12, lorsque le produit de programme d'ordinateur est exécuté dans l'unité informatique.
